(19) Europäisches Patentamt
European Patent Office
Office européen des brevets

(11) Publication number: **0 462 060 A2**

# (12) EUROPEAN PATENT APPLICATION

(21) Application number: **91810422.5**

(51) Int. Cl.$^5$: **C07C 229/56,** C08G 59/50

(22) Date of filing: **04.06.91**

(30) Priority: **13.06.90 GB 9013239**

(43) Date of publication of application:
**18.12.91 Bulletin 91/51**

(84) Designated Contracting States:
**BE DE ES FR GB IT NL SE**

(71) Applicant: **CIBA-GEIGY AG**
**Klybeckstrasse 141**
**CH-4002 Basel (CH)**

(72) Inventor: **Stowell, Jonathan Andrew**
**39 Impala Drive**
**Cherry Hinton, Cambridge CB1 4XJ (GB)**

(54) **Curable composition.**

(57) A heat-curable composition comprising:
a) an epoxide resin; and
b) as curing agent, at least one aromatic amine derivative having the formula I:

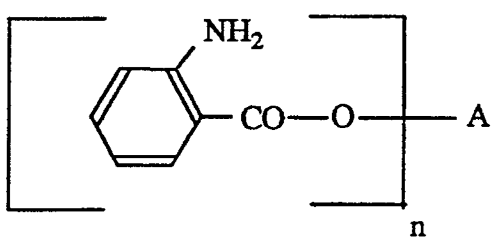

I

in which A is an n-valent aromatic residue and n is a number ranging from 2 to 400, and A is connected to a moiety CO-O- via aromatic carbon atoms.

Most of the compounds of formula I are new.

EP 0 462 060 A2

The present invention relates to heat-curable epoxy resin compositions containing, as curing agents, aromatic amines, some of which are new compounds.

It is known that epoxide resins, i.e. substances containing, on average, more than one 1,2-epoxide group per molecule, may be cured by reaction with various classes of compounds, to form crosslinked, infusible, insoluble products having valuable properties e.g. as castings.

We have found that by using, as curing agents, certain aromatic amine derivatives, epoxy resin compositions are obtained which have good storage stability at ambient temperature and which when cured, have high glass transition temperatures.

Accordingly, the present invention provides a heat-curable composition comprising:

a) an epoxide resin; and

b) as curing agent, at least one aromatic amine derivative having the formula I:

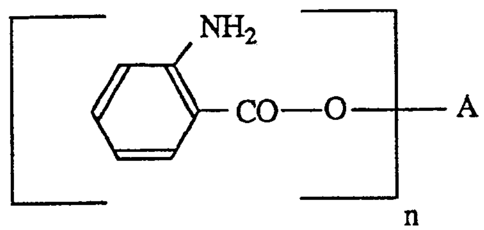

I

in which A is an n-valent aromatic residue and n is a number ranging from 2 to 400, and A is connected to a moiety CO-O- via aromatic carbon atoms.

The epoxide component a) may be any epoxy resin but is preferably one which contains at least two groups of formula II:

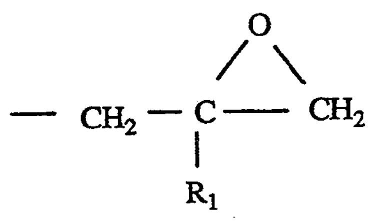

II

in which $R_1$ is hydrogen or methyl, the group II being directly attached to one or more atoms of oxygen, nitrogen or sulphur.

Examples of such preferred resins a) include polyglycidyl and poly(beta-methylglycidyl) esters, obtained by reacting a compound containing two or more carboxylic acid groups per molecule, with epichlorohydrin, glycerol dichlorohydrin or beta-methyl-epichlorohydrin, in the presence of an alkali. Such polyglycidyl esters may be derived from aliphatic carboxylic acids e.g. oxalic acid, succinic acid, glutaric acid, adipic acid, pimelic acid, suberic acid, azelaic acid, sebacic acid or dimerized or trimerized linoleic acid; from cycloaliphatic polycarboxylic acids such as tetrahydrophthalic acid, 4-methyltetrahydrophthalic acid, hexahydrophthalic acid and 4-methylhexahydrophthalic acid; and from aromatic polycarboxylic acids e.g. phthalic acid, isophthalic acid and terephthalic acid.

Further examples are polyglycidyl and poly(betamethylglycidyl) ethers which are obtainable by reacting a compound containing at least two free alcoholic hydroxyl and/or phenolic hydroxyl groups per molecule, with the appropriate epichlorohydrin, under alkaline condition, or in the presence of an acidic catalyst, with subsequent treatment with alkali. The hydroxyl group - containing reactant may be, e.g. an acyclic alcohol such as ethylene glycol, diethylene glycol or higher poly(oxyethylene)-glycols, propane-1,2-dioland poly(oxypropylene) glycols, propane-1,3-diol,butane-1,4-diol, poly(oxytetramethylene)glycols, pentane-1,5-diol, hexane-1,6-diol,hexane-2,4,6-triol, glycerol 1,1,1-trimethylolpropane, pentaerythritol, sorbitol, and polyepichlorohydrins; from cycloaliphatic alcohols such as resorcitol, quinitol, bis (4-hydroxycyclohexyl)methane, 2,2-bis-(4-hydroxymethyl)cyclohex-3-ene; and from alcohols having aromatic nuclei such as N,N-bis(2-hydroxyethyl)aniline and p,p[1]-bis(2-hydroxylethylamino) diphenyl methane. The hydroxyl-group containing reactant may also be a mononuclear phenol such as resorcinol and hydroquinone, or a polynuclear phenol such as bis(4-hydroxyphenyl) methane, 4,4[1]-dihydroxydiphenyl, bis(4-hydroxyphenyl)sulphone, 1,1,2,2-tetrakis-(4-hydroxyphenyl)ethane, 2,2-bis(4-hydroxyphenyl)propane, 2,2-bis(3,5-di bromo-4-hydroxyphenyl)-propane and novolaks formed from aldehydes such as formaldehyde, acetaldehyde, chloral and furfuraldehyde, with phenols

such as phenol itself, and phenol ring-substituted with chlorine atoms, or $C_1$-$C_9$ alkyl groups, e.g. 4-chlorophenol, 2-methylphenol and 4-tert. butylphenol.

Poly(N-glycidyl) compounds include, e.g., those obtained by dehydrochlorination of the reaction products of epichlorohydrin with amines containing at least two amino hydrogen atoms, such as aniline, n-butylamine, bis(4-aminophenyl) methane, m-xylylenediamine, and bis(4-methylaminophenyl)-N,N$^1$-diglycidyl derivatives of cylic alkylene ureas, such as ethyleneurea and 1,3-propyleneurea, and of a hydantoin such as 5,5-dimethyl hydantoin.

Examples of poly (S-glycidyl) compounds are di-S-glycidyl derivatives of dithiols such as ethane- 1,2-dithiol and bis(4-mercaptomethylphenyl) ether.

Epoxide resins having the 1,2-epoxide groups attached to different kinds of hetero atoms may be employed, e.g. the N,N,O-triglycidyl derivative of 4-aminophenol, the glycidyl ether-glycidyl ester of salicylic acid, N-glycidyl-N$^1$-(2-glycidyloxypropyl)-5,5-dimethyl hydantoin and 2-glycidyloxy- 1,3-bis(5,5-dimethyl-1-glycidyl hydantoin-3-yl)propane.

If desired, a mixture of epoxide resins may be used.

Preferred epoxide resins are liquids, and include polyglycidyl ethers, polyglycidyl esters, N,N$^1$-diglycidyl hydantoins and poly(N-glycidyl) derivatives of aromatic amines. Specific preferred resins are polyglycidyl ethers of 2,2-bis(4-hydroxyphenyl) propane, of bis(4-hydroxyphenyl)methane or of a novolak formed from formaldehyde and phenol optionally substituted in the ring by one chlorine atom or by one $C_1$-$C_9$ alkyl group and having a 1,2-epoxide content of at least 0.5 equivalent per kilogram, bis(4-(diglycidylamino)phenyl] methane or p-(diglycidylamino)phenyl glycidyl ether.

In the compounds of formula 1, component b) of the compositions of the present invention, n ranges from 2 to 400. When n is 2, A is a divalent aromatic residue which may be derived from e.g., o-, m- or p-phenylene; o-, m- or p-diphenylene; diphenyl methane; 2,2-diphenyl-propane; or diphenyl sulphone. When n is up to 400 A is a polyvalent aromatic residue which may be a residue of formula:

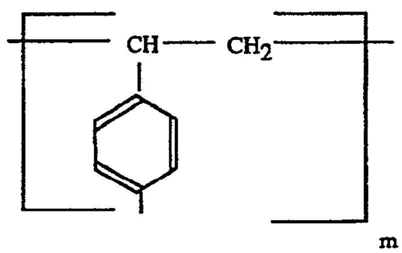

in which m is an integer ranging from 1 to 400. When n is 4, A is a tetravalent aromatic residue which may be a benzene-1,2,4,5-tetrayl residue. When n is 8, A is an octavalent aromatic residue (calixarene) having the formula:

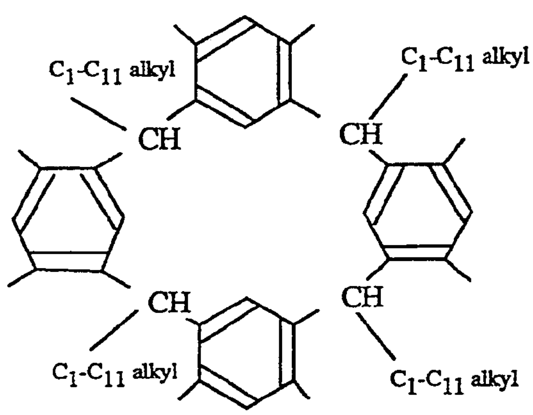

the preferred $C_1$-$C_{11}$ alkyl groups being $C_1$-$C_4$ alkyl groups, especially methyl groups.

A may also be an aromatic residue derived by removal of hydroxyl functions from polyphenolic products obtained by reacting aldehydes with phenols. Typical and important examples of such polyphenols are commercially available products from formaldehyde and phenols such as phenol itself and o, p, or m-cresol. In these instances, n would typically be an average number in the range of 2 - 20.

The compositions of the present invention usually contain the amine derivative of formula I in an amount ranging from 0.4 to 1.2, preferably 0.8 to 1.0 equivalents of active amino hydrogen, per epoxy equivalent.

Most of the compounds of formula I are believed to be new compounds. Some of the compounds of formula 1, however, are known. Thus, in German Laid-open specification No. 2052755, there are described compounds, useful as UV light absorbers, and having the formula:

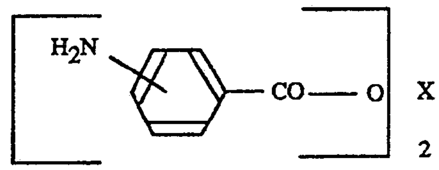

in which X is phenylene or a biphenyl bridging member of formula:

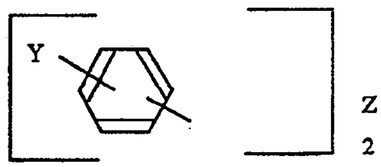

in which Y is hydrogen or halogen and Z is a sulphonyl group or a $C_3$-$C_8$ alkylidene group.

Accordingly, the present invention also provides compounds having the formula IA:

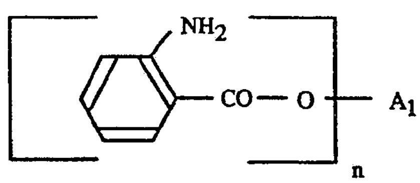

IA

wherein $A_1$ is a n-valent aromatic residue and n is up to 400 provided that, when n is 2, $A_1$, is not phenylene or a biphenyl bridging member of formula:

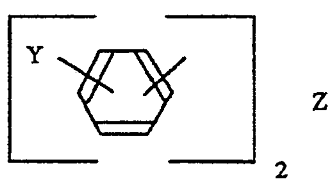

in which Y is hydrogen or halogen or Z is a sulphonyl group or a $C_3$-$C_8$ alkylidene group.

In special compounds of formula IA n is 2 and $A_1$, is o-, m- or p- diphenylene.

Preferred are compounds of formula IA in which n is up to 400 and $A_1$ is a residue of formula:

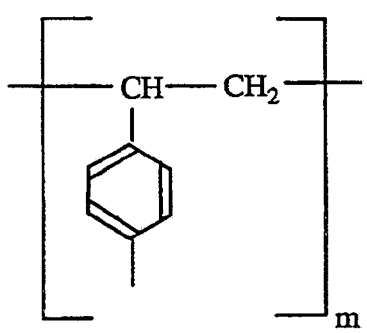

in which m is an integer ranging from 1 to 400.

Preferred compounds of formula IA are also those in which n is 4 and $A_1$ is a 1,2,4,5-phenylene residue.

Particularly preferred are also those compounds of formual IA in which n is 8 and $A_1$ is a calixarene residue of formula:

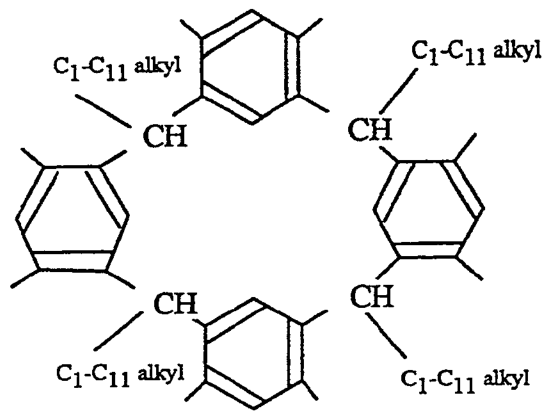

especially those in which each $C_1$-$C_{11}$ alkyl group is methyl.

A further object of the invention are compounds of formula IA in which $A_1$ is a residue obtained by removing hydroxyl groups from a phenol-aldehyde resin, especially if the aldehyde is formaldehyde and/or the phenol is phenol itself or a cresol.

The compounds of formula I may be prepared in the manner disclosed, e.g. in German Laid-open specification No. 2052755, by reacting a compound of formula:

$$A_1\,(OH)_n$$

with isatoic anhydride, in the presence of a strong base.

Specific examples of preferred compounds of formula I include
2,2[1]-bis-[4-(2-aminobenzyloxy)-phenyl]propane; bis [p-(2-aminobenyloxy)-phenyl] sulphone; 4,4[1]-biphenol, bis(2-aminobenzoate);
2,8,14,20-tetramethylpentacyclo[19.3.1.1$^{3,7}$.1$^{9,13}$.1$^{15,19}$]octacosa-1(25),3,5,7,(28,9,11,13(27),15,17,19,(26),2 1,23-dodecaene-4,6,10,12,16,18,22,24-octol, octa(2-aminobenzoate);
1,3-benzenediol bis(2-aminobenzoate); poly[4-(2-aminobenzoyloxy)styrene];
1,4-benzenediol bis(2-aminobenzoate); and 1,3,5-benzenetriol, tris(2-aminobenzoate).

Of these preferred compounds 2,2[1]-bis-[4-(2-aminobenzyloxy)-phenyl]propane and 1,3-benzenediol, bis(2-aminobenzoate) have been specifically disclosed in German Laid-open specification No. 2052755.

The heat-curing of the epoxide resin compositions of the present invention may be promoted by the inclusion of an accelerator. Suitable accelerators incude dicyandiamide, carboxylic acid hydrazides, succinimide, cyanoacetamine, 1-cyano-3-($C_1$-$C_3$-alkyl) guanidines, imidazoles, and salts of carboxylic acids with tertiary amines. Normally, the accelerator will be present in an amount of 2 to 50 parts by weight, per 100 parts by weight of the curing agent of formula I.

The new compositions according to the present invention may also contain suitable plasticizers such as dibutyl phthalate or dioctyl phthalate; inert diluents e.g. tars and bitumen; and so-called reactive diluents, especially monepoxides e.g. n-butyl glycidyl ether, iso-octyl glycidyl ether, phenyl glycidyl ether, cresyl glycidyl ether, glycidyl esters of mixed tertiary, aliphatic monocarboxylic acids, glycidyl acrylate and glycidyl methacrylate. The compositions may also contain additives such as fillers; reinforcing materials; polymeric toughening agent such as polyether sulphones, phenoxy resins and butadiene-acrylonitrile rubbers; colouring matter, flow control agents; flame retardants; and mould lubricants. Suitable extenders, fillers and reinforcing materials are, e.g., glass fibres, carbon fibres, fibres of aromatic polyamides, ballotini, mica, quartz flour, calcium carbonate, cellulose, kaolin, wollastonite, colloidal silica having a large specific surface area, powdered poly (vinyl chloride) and powdered polyolefin hydrocarbons such as polyethylene and polypropylene.

The compositions of the present invention are useful as laminating resins, impregnating and casting resins, powder coatings, moulding compositions, putties and sealing compounds, potting and insulating compounds for the electrical industry, but especially as casting resins.

The compositions of the invention are conveniently cured by heating them at a temperature in the range of from 150°C to 200°C. Usually, heating for 30 to 120 minutes suffices to achieve curing.

It is, however, sometimes necessary to post-cure materials at more elevated temperatures to achieve

optimum properties.

The following Examples further illustrate the present invention. Examples 1 and 5 relate to the preparation of known compounds which are useful in the preparation of new compositions of the present invention.

EXAMPLE 1

Isatoic anhydride (32.6g, 0.2 moles), Bisphenol A (22.8g, 0.1 moles), sodium hydroxide (0.3g) and dioxan (300g) are stirred and heated for 6 hours at 80°C. The mixture is cooled and poured into 1 litre of water. The precipitate is filtered and washed. The reaction yields 39.1 g (84% of theory) of 2,2[1]-bis- [4-(2-aminobenzoyloxy)-phenyl] propane.
Melting point 194°C.

ANALYSIS: Found: C,73.16; H,5.92; N,5.73%. $C_{29} H_{26} O_4 N_2$ requires C, 74.69; H,5.62; N,6.01%. IR (KBr disc): 3380 and 3480$^{-1}$ ($NH_2$); 1705cm$^{-1}$ (C=O); NMR ($CDC1_3$): 1.72(s-6H); 5.78(broad s-4H); 6.7-6.75(m-4H); 7.08-7. 12(q-4H); 7.26-7.37(m-6H); 8.06-8.1(q-2H).

EXAMPLE 2

Example 1 is repeated using, as starting materials, isatoic anhydride (32.6g, 0.2 moles), Bisphenol S (25g, 0.1 moles), sodium hydroxide (0.3g) and dioxan (300g). The reaction yields 26.2g (54% of theory) of bis(p-[1-aminobenzyloxy]-phenyl) sulphone. Melting point 240°C.

ANAYSIS: Found: C,63.29; H,4.38; N,5.50%. $C_{26}H_{20}O_6S N_2$ requires C,63.93; H,4.13; N,5.73%. IR (KBr disc): 3360 and 3460cm$^{-1}$ ($NH_2$); 1700cm$^{-1}$ (C=O): NMR ($CDC1_3$): 5.78(broad s-4H); 6.7-6.8(m-4H); 7.2-7.4(m-6H); 8.0-8.2(m-6H).

EXAMPLE 3

Example 1 is repeated using, as starting materials, isatoic anhydride (32.6g, 0.2 moles), 4-4[1] biphenol (18.6g, 0.1 moles), sodium hydroxide (0.3g) and dioxan (300g). The reaction yields 37.3g (88% of theory) of 4-4[1] biphenol, bis(2-aminobenzoate). Melting point 287°C.

ANALYSIS: Found: C,72.39: H,4.81; N,6.46%. $C_{26} H_{20} O_4 N_2$ requires C,73.57; H,4.75; N,5.60%. IR (KBr disc): 3360 and 3460 cm$^{-1}$ ($NH_2$); 1685cm$^{-1}$ (C=O): NMR (DMSO-$d_6$): 6.6-6.9(m-8H); 7.3-7.4(d-6H); 7.7-7.8(d-4H); 7.9-8.0(d-2H).

EXAMPLE 4

Example 1 is repeated using, as starting materials, isatoic anhydride (65.25g, 0.4 moles), 2,8,14,20-tetramethylpentacyclo[19.3.1.$^{3,7}$.1$^{9,13}$.1$^{15,19}$] octacosa-(25),3,5,7(28),9,11,13(27),15,17,19(26),21,23-dodecaene- 4,6,10,12,16,18,22,24-octol (27.2g, 0.05 moles), sodium hydroxide (0.3g) and dioxan (300g). The reaction yields 54.4g (75% of theory) of 2,8,14,20-tetramethylpentacyclo [19.3.1.1$^{3,7}$.1$^{15,19}$] octacosa- 1 (25),3,5,7(28),9.11,13(27),15,17,19(26),21,23-dodecaene-4,6,10,12,16,18,22,24 ,-octol, octa(2-aminobenzoate). Melting point 240°C.

ANALYSIS: Found: C,70.12; H,4.90; N,7.20%. $C_{88} H_{72} O_{16} N_8$ requiresC,70.57; H,4.84; N,7.48%. IR (KBr disc): 3380 and 3500cm$^{-1}$ ($NH_2$); 17 10cm$^{-1}$ (C=O): NMR (acetone-$d_6$): 1.6(d-12H); 2.9(broad s-16H); 4.5(q-4H); 6.4-7.9(m-40H).

EXAMPLE 5

Example 1 is repeated using, as starting materials, isatoic anhydride (65.2g, 0.4 moles), resorcinol (22.0g, 0.2 moles), sodium hydroxide (0.3g) and dioxan (300g). The reaction yields 40.5g (58% of theory) of 1,3-benzenediol, bis(2-aminobenzoate). Melting Point 140°C.

ANALYSIS: Found: C,68.87; H,4.80; N,7.93%. $C_{20} H_{16} O_4 N_2$ requires C,68.96; H,4.63; N,8.04%. IR (KBr disc): 3390 and 3500cm$^{-1}$ ($NH_2$; 1700cm$^{-1}$ (C=O): NMR (Acetone-$d_6$): 6.5(broad s-4H); 6.63-6.69(m-2H); 6.86-6.90(m-2H); 7.2-7.5(m-6H); 8.0(q-2H).

EXAMPLE 6

Example 1 is repeated using, as starting materials, isatoic anhydride (32.6g, 0.2 moles), poly(4-hydroxy styrene) (24.0g, 0.2 moles), sodium hydroxide (0.3g) and dioxan (300g). The reaction yields 42.0g (88% of

theory) of Poly(4-(2-aminobenzoyloxy)styrene). Melting point 170°C.

ANALYSIS: Found: C,73.76; H,5.75; N,5.67%. $C_{15}$ $H_{13}$ $O_2$ N requires C,75.29; H,5.47; N,5.85%. IR (KBr disc): 3375 and 3480$cm^{-1}$ ($NH_2$; 1700$cm^{-1}$ (C=O): NMR (Acetone-$d_6$):

EXAMPLE 7

Example 1 is repeated using, as starting materials, isatoic anhydride (65.2g, 0.4 moles), hydroquinone (22.0g, 0.2 moles), sodium hydroxide (0.3g) and dioxan (300g). The reaction yields 49.3g (70% of theory) of 1,4-benzenediol,bis(2-aminobenzoate). Melting point 205°C.

ANALYSIS: Found: C,68.29; H,4.84; N,7.98%. $C_{20}$ $H_{16}$ $O_4$ $N_2$ requires C,68.96; H,4.63; N,8.04%. IR (KBr disc): 3380 and 3480$cm^{-1}$ ($NH_2$; 1705$cm^{-1}$ (C=O): NMR ($CDC1_3$): 5.8(broad s-4H); 6.7-6.8(m-4H); 7.2-7.4(m-6H); 8.1-8.2(m-2H).

EXAMPLE 8

Example 1 is repeated using, as strating materials isatoic anhydride (48.9g, 0.3 moles), phloroglucinol (12.6g, 0.1 moles), sodium hydroxide (0.3g) and dioxan (300g). The reaction yields 44.1g (91% of theory) of 1,3,5-benzenetriol,tris(2-aminobenzoate). Melting point 170°C.

ANALYSIS: Found: C,66.90; H,4.52; N,8.54%. $C_{27}$ $H_{21}$ $O_6$ $N_3$ requires C,67.07; H,4.38; N,8.69%. IR (KBr disc): 3320, 3390, 3415 and 3495$cm^{-1}$ ($NH_2$); 1710$cm^{-1}$ (C=O): NMR ($CDC1_3$): 5.77(broad s-6H); 6.6-6.8(m-7H); 7. 1 (s-2H); 7.3-7.4(m-3H); 8.0-8.1(m-3H).

EXAMPLES 9 TO 16

In stoichiometric mixes with diglycidyl ether of bisphenol A having an epoxide content of 5.3 equivs./Kg;, cured at 175°C for 3 hours followed by 2 hours at 250°C the following $T_g$'s are obtained:

| EXAMPLE | AMINE | $T_g$(°C) |
|---|---|---|
| 9 | 2,2-Bis-[4-(2-aminobenzoyloxy-phenyl] propane (Product of Example 1) | 125 |
| 10 | bis(p-[2-aminobenzyloxy]-phenyl) sulphone (Product of Example 2) | 140 |
| 11 | 4-4$^1$ biphenol, bis(2-aminobenzoate) (Product of Example 3) | 128 |
| 12 | 2,8,14,20-tetramethylpentacyclo[19.3.1.1$^{3,7}$.1$^{15,19}$] octacosa-1(25),3,5,7(28),9,11,13(27),15,17, 19(26),21,23-dodecaene-4,6,10,12,16,18,22,24-octol, octa(2-amino-benzoate) (Product of Example 4) | 178 |
| 13 | 1,3-benzenediol,bis(2-aminobenzoate) (Product of Example 5) | 128 |
| 14 | Poly(4-(2-aminobenzolyloxy)styrene) (Product of Example 6) | 158 |
| 15 | 1,4-benzenediol,bis(2-aminobenzoate) (Product of Example 7) | 120 |
| 16 | 1,3,5-benezenetriol,tris(2-aminobenzoate) (Product of Example 8) | 141 |

EXAMPLES 17 AND 18

In mixes with N,N,N$^1$,N$^1$-tetraglycidyl 4,4$^1$diaminodiphenylmethane, cured at 175°C for 3 hours, followed by 2 hours at 250°C, the following $T_g$'s are obtained:

| EXAMPLE | AMINE | $T_g$(°C) |
|---|---|---|
| 17 | bis(p-[2-aminobenzyloxy]-phenyl) sulphone (Product of Example 2) | 225 |
| 18 | 1,3,5-benzenetriol, tris(2-aminobenzoate) (Product of Example 8) | 234 |

EXAMPLES 19 TO 26

In stoichiometric mixes with diglycidyl ether of bisphenol A having an epoxide content of 5.3 equivs. per Kg maintained at 25°C the following storage lives are obtained:

| EXAMPLE | AMINE | STORAGE LIFE (weeks) |
|---|---|---|
| 19 | 2,2'-Bis-[4-(2-aminobenzoyloxy)-phenyl] propane (Product of Example 1) | 8 |
| 20 | bis(p-[2-aminobenzyloxy]-phenyl) sulphone (Product of Example 2) | 5 |
| 21 | 4-4' biphenol, bis(2-aminobenzoate) (Product of Example 3) | 5 |
| 22 | 2,8,14,20-tetramethylpentacyclo[19.3.1.$1^{3,7}$.$1^{15,19}$] octacosa-1(25),3,5,7(28),9,11,13(27),15,17, 29(26),21,23-dodecaene-4,6,10,12,16,18,22,24-octol, octa(2-aminobenzoate) (Product of Example 4) | 1.5 |
| 23 | 1,3-benzenediol,bis(2-aminobenzoate) (Product of Example 5). | 7 |
| 24 | Poly(4-(2-aminobenzoyloxy)styrene (Product of Example 6) | 4 |
| 25 | 1,4-benzenediol, bis(2-aminobenzoate) (Product of Example 7) | 4 |
| 26 | 1,3,5-benzenetriol,tris(2-aminobenzoate) (Product of Example 8) | 3 |

EXAMPLES 27 AND 28

Castings, made from stoichiometric mixes with diglycidyl ether of bisphenol A having an epoxide content of 5.3 equivs. per Kg. cured at 175°C for 3 hours followed by 2 hours at 250°C and placed in water for 1000 hours, have the following water uptake:

| EXAMPLE | AMINE | WATER UPTAKE (Wt%) |
|---|---|---|
| 27 | 2,8,14,20-tetramethylpentacyclo[19.3.1.$1^{3,7}$.$1^{9,13}$.$1^{15,19}$]octacosa-1(25),3,5,7(28),9,11,13(27),15,17,19,(26),21,23-1.5-dodecaene-4,6,10,12,16,18,22,24-octol, octa(2-aminobenzoate) (Product of Example 4) | 1.66 |
| 28 | 1,3,5-benzenetriol,tris(2-aminobenzoate) (Product of Example 8) | 2.18 |

**Claims**

1. A heat-curable composition comprising:
   a) an epoxide resin; and
   b) as curing agent, at least one aromatic amine derivative having the formula I:

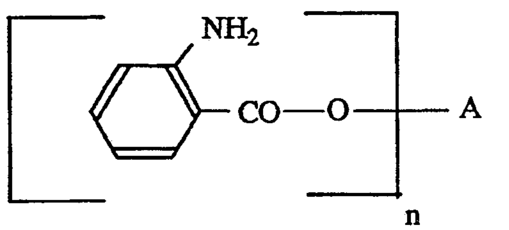

I

in which A is an n-valent aromatic residue and n is a number ranging from 2 to 400, and A is connected to a moiety CO-O- via aromatic carbon atoms.

2. A composition according to claim 1 in which the epoxide component a) contains at least two groups of formula II:

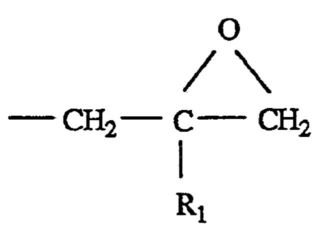

II

in which $R_1$ is hydrogen or methyl, the group II being directly attached to one or more atoms of oxygen, nitrogen or sulphur.

3. A composition according to claim 2 in which the epoxide component a) is a ' polyglycidyl or poly(beta-methylglycidyl) ester or ether.

4. A composition according to claim 2 in which the epoxide component a) is a poly(N-glycidyl) compound or a poly(S-glycidyl) compound.

5. A composition according to claim 2 in which the epoxide component a) is a liquid and is a polyglycidyl ether, a polyglycidyl ester, an N,N[1]-diglycidyl hydantoin or a poly(N-glycidyl) derivative of an aromatic amine.

6. A composition according to any of claims 1 to 5 in which in the compounds of formula I, n is 2 and A is derived from o-, m- or p- phenylene; o-, m- or p- diphenylene; diphenyl methane; 2,2-diphenyl propane; or diphenyl sulphone.

7. A composition according to any of claims 1 to 5 in which n is up to 400 and A is a residue of formula:

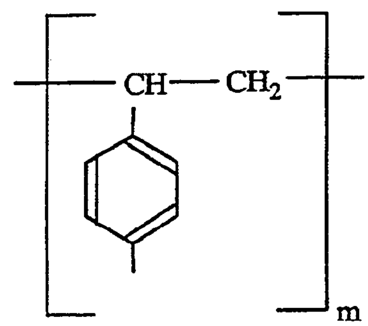

in which m is a positive integer of 1 to 400.

8. A composition according to any of claims 1 to 5 in which n is 4 and A is a benzene- 1,2,4,5-tetrayl residue.

9. A composition according to any of claims 1 to 5 in which n is 8 and A is a calixarene residue having the formula:

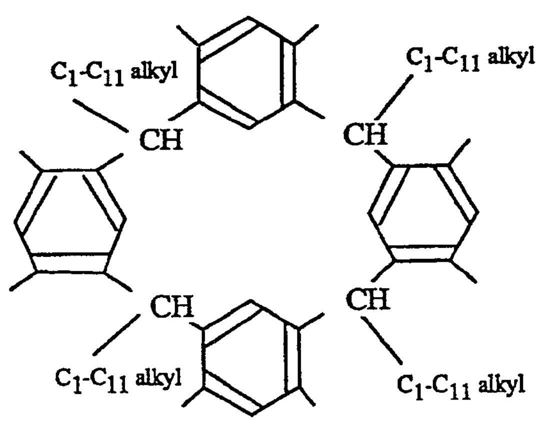

10. A composition according to any of claims 1 to 5 in which A is the polyaromatic residue derived by removal of phenolic hydroxyl groups from a reaction product of a phenol and an aldehyde.

11. A composition according to claim 1 in which the compound of formula I is present in an amount of 0.4 to 1.2, preferably of 0.8 to 1.0, equivalents of active amino hydrogen per epoxy equivalent.

12. A composition according to claim 1 in which an accelerator is present.

13. A process for curing an epoxide resin comprising forming a composition according to claim 1; and then heating the composition until the epoxide resin is cured.

14. A process according to claim 13 in which the composition is heated at a temperature ranging from 150°C to 200°C for 30 to 120 minutes.

**15.** A compound having the formula IA:

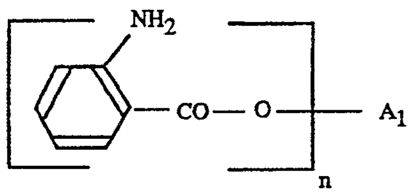

in which $A_1$, is an n-valent aromatic residue and n is up to 400 provided that, when n is 2, $A_1$ is not phenylene or a biphenyl bridging member of formula:

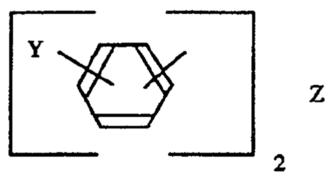

in which Y is hydrogen or halogen and Z is a sulphonyl group or a $C_3$-$C_8$ alkylidene group.

**16.** A compound of formula IA according to claim 15 in which n is 2 and $A_1$, is o-, m- or p- diphenylene.

**17.** A compound of formula IA according to claim 15 in which n is up to 400 and $A_1$ is a residue of formula:

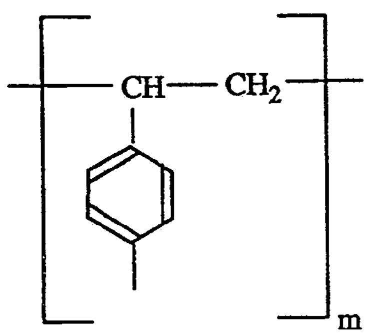

in which m is an integer ranging from 1 to 400.

**18.** A compound of formula IA according to claim 15 in which n is 4 and $A_1$ is a 1,2,4,5-phenylene residue.

**19.** A compound of formula IA according to claim 15 in which n is 8 and $A_1$ is a calixarene residue of formula:

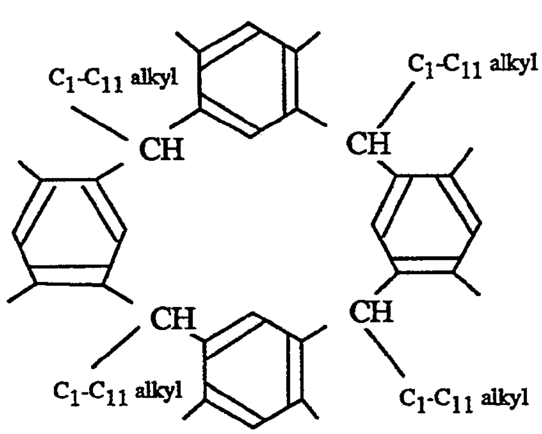

**20.** A compound of formula IA in which $A_1$ is a residue obtained by removing hydroxyl groups from a phenol-aldehyde resin.